**Europäisches Patentamt**

**European Patent Office**

**Office européen des brevets**

(19)

(11) Publication number : **0 427 998 B1**

(12)

# EUROPEAN PATENT SPECIFICATION

(45) Date of publication of patent specification :
05.05.93 Bulletin 93/18

(51) Int. Cl.⁵ : **A61K 37/30, A61K 9/48, A61K 9/02**

(21) Application number : **90120678.9**

(22) Date of filing : **29.10.90**

(54) A pharmaceutical composition for rectal administration of calcitonin.

(30) Priority : **16.11.89 IT 2239889**

(43) Date of publication of application :
**22.05.91 Bulletin 91/21**

(45) Publication of the grant of the patent :
**05.05.93 Bulletin 93/18**

(84) Designated Contracting States :
**BE CH DE ES FR IT LI**

(56) References cited :
**EP-A- 0 308 725**
**WPIL, AN=81-90229D [49], Derwent Publications Ltd, London, GB**
**WPIL, AN=81-90229D [49], Derwent Publications Ltd, London, GB**

(73) Proprietor : **PHIDEA S.p.A.**
**Viale Renato Serra, 6**
**I-20148 Milan (IT)**

(72) Inventor : **Ceschel, Giancarlo**
**Piazza IV Febbraio 14**
**I-20145 Milan (IT)**

(74) Representative : **Giambrocono, Alfonso, Dr. Ing. et al**
**Ing. A. Giambrocono & C. S.r.l. Via Rosolino Pilo 19/B**
**I-20129 Milano (IT)**

EP 0 427 998 B1

## Description

The present invention relates to a pharmaceutical composition, having good absorption and stability characteristics, for the rectal administration of a calcitonin as the active ingredient.

The calcitonin is a simple polypeptide comprising 32 aminoacids that in mammals, such as rat, guinea pig, rabbit, dog, sheep, goat, pig, cow, monkey, as well as in man, is secreted by thyroid, and in fishes (salmon, trout, eel, and carp,), in birds, such as the hen, and in amphibians, is secreted by the branchial glands. The aminoacid sequence, as well as their configuration in calcitonin of the different animal species, differs in a more or less considerable way, the physiological functions of the hormone remaining nevertheless essentially unchanged. Said functions seem to concern the lowering of calcium ion and inorganic phosphorous level in blood by means of an action on bones, intestine, and kidneys.

In particular, calcitonin prevents the salts from being resorbed by the bone tissue: said effect preventing an excessive loss of calcium from the bones during pregnancy and delivery from occurring, as well as calcium from being removed from the bones of old animals. In therapy, calcitonin is used in the treatment of the Paget disease, in osteoporosis, in hypercalcemia, and generally in all the diseases related with an excessive calcium content in blood.

The term "calcitonin" indicates in the present invention a calcitonin of natural origin, such as salmon, pig, eel or human calcitonin, as well as the synthetic products having the same structure and pharmacological activity; all these products are commercially available and widely described in the scientific literature.

This polypeptide is usually administered by means of injectable preparations that can cause strong pain to the patient and hinder an easy self-medication in case of long term treatments. In view of the above, many researches have been carried out to find new routes of administration for this polypeptide that would not exhibit the drawbacks of the injectable form.

The result of said researches has brought to the discovery according to which calcitonin can be administered by alternative routes that eliminate the drawbacks of the parenteral routes, such as the limited use practicality, the need of operating in a sterile environment during the packaging processes, pain accompanying the administration, while producing similar effects as those of the parenteral route, especially of the intramuscular route, or anyway quite appreciable effects.

The rectal administration route of calcitonin could be one of such alternative routes, as the active ingredient is resorbed without undergoing degradation by the digestive apparatus. But the studies carried out up to now on peptides show that said compounds are not easily absorbed by the rectal wall and, especially calcitonins, show a low pharmacological activity when administered by the rectal route. In patent applications EP-A-0037943, JP-81/118013, JP-81/122309, NL-8600299, FR 2.623.090 and in US patent no. 4,609,640, preparations for the rectal administration of a calcitonin are described in which the active ingredient dosage, even though a absorption adjuvant is present, is generally higher than the dosage used in pharmaceutical compositions for intramuscular administration.

Also in Journal of Pharmaceutical Sciences (1984), 73 (10), 1366-8, pharmaceutical composition is described containing calcitonin in a polyacrylic acid gel base; the data in the above-mentioned paper indicate that a dosage about 35 times as high as the intramuscular calcitonin dosage is needed to obtain the same therapeutical results. The patent application IT-22031/87 describes a composition that is suitable for the rectal administration of a calcitonin without the use of any absorption adjuvant. More precisely, in the above-mentioned patent application the active ingredient is mixed with polyethylene glycol or polypropylene glycol as the vehicle, and said composition is used for the preparation of gelatin capsules; but the employed vehicle has the drawback of being hygroscopic and withdrawing water from the gelatine envelop, thereby altering the preparation stability.

It would therefore be desirable to obtain pharmaceutical compositions that can be used by rectal administration, in which the pharmacologically effective dosage of calcitonin is comparable with the dosages used in compositions for parenteral and/or intravenous administration, and in which the active ingredient contained in the gelatine capsules is stable in time.

There has now been surprisingly found a pharmaceutical composition, having good absorption and stability characteristics, for the rectal administration of a calcitonin.

In particular, the present invention relates to a pharmaceutical composition for the rectal administration comprising a calcitonin in a suitable oily vehicle that does not damage the polypeptide contained in gelatine capsules, thereby imparting the preparation a very good stability, said oily vehicle being also in a measure of giving rise to an appreciable absorption of calcitonin through the rectal mucose.

The present invention relates therefore to a pharmaceutical composition useful for the rectal administration by means of soft gelatine capsules comprising 50-600 I.U. of a calcitonin dispersed in about 400-2000 mg of an oily vehicle, said oily vehicle consisting of a mixture comprising 40-90% of a liquid paraffin and 10-60% of

EP 0 427 998 B1

at least one hydrocarbon chosen between soft and/or solid paraffin, preferably in the presence of about 10-50 mg of at least one non-ionic surfactant chosen from the group of polysorbates, such as Polysorbate 80® or Span 80®.

For the preparation of the pharmaceutical composition which is the object of the present invention, there is preferably used a salmon calcitonin, more advantageously in a quantity of from 50 to 300 I.U., dispersed in about 900 mg of an oily vehicle. Said vehicle preferably comprises a mixture of 65% liquid paraffin and 35% solid paraffin.

In the above-described pharmaceutical composition, also 10-50 mg of at least one non-ionic surfactant, as they have been described hereinabove, can be advantageously used.

Said formulations can be used in the preparation, according to known techniques, of soft gelatine capsules used in unit dosage forms. For the preparation of a pharmaceutical composition of the present invention, the prescribed dosages of calcitonin, the oily vehicle and possibly a surfactant are used. After mixing with a suitable stirrer, the mixture thus obtained is filled into soft gelatine capsules containing from about 400 to about 2000 mg, preferably from about 900 to about 1200 mg, of the above-described pharmaceutical composition. The soft gelatine envelope can be suitably prepared according to the method described in the European patent applications EP-A-120248 and EP-A-121321.

The term "international unit" (I.U.) as used in the present invention is as a defined by World Health Organization as related to the International Reference Preparations for the biological titration of calcitonins of the various species (man, pig, salmon and eel calcitonin), distributed by the National Institute for Biological Standards and Control (NIBSAC), South Mimms, Potters Bar, Hertfordshire, EN6 30G, United Kingdom.

The following non limiting examples illustrate the present invention.

EXAMPLE 1

For 1000 soft gelatine capsules containing about 900 mg each, the following composition is prepared:
Ingredients:

| | |
|---|---|
| Liquid paraffin | 585 g |
| Soft paraffin | 315 g |
| Calcitonin | 55000 I.U. |

Preparation:

The oily vehicle is first prepared by mixing the soft paraffin with the liquid paraffin. The calcitonin is then added to the oily vehicle, stirring until the mixture is homogeneous. The mass is then filled into the capsules.

EXAMPLE 2

For 1000 soft gelatine capsules containing about 900 mg each, the following composition is prepared:
Ingredients:

| | |
|---|---|
| Liquid paraffin | 585 g |
| Soft paraffin | 315 g |
| Polysorbate 80® | 10g |
| Calcitonin | 55000 I.U. |

Preparation:

The oily vehicle is first prepared by mixing the soft paraffin with the liquid paraffin, followed by adding the surfactant. The calcitonin is then added to the oily vehicle, stirring until the mixture is homogeneous. The mass is then filled into the capsules.

EXAMPLE 3

For 1000 soft gelatine capsules containing about 900 mg each, the following composition is prepared:
Ingredients

| | |
|---|---|
| Liquid paraffin | 555.7 g |
| Soft paraffin | 299.3 g |
| Polysorbate 80® | 22.5 g |
| Span 80® | 22.5 |
| Calcitonin | 55000 I.U. |

Preparation:

The oily vehicle is first prepared by mixing the soft paraffin with the liquid paraffin, followed by adding the surfactants. The calcitonin is then added to the oily vehicle, stirring until the mixture is homogeneous. The

3

mass is then filled into the capsules.

EXAMPLE 4

For 1000 soft gelatine capsules containing about 400 mg each, the following composition is prepared:

Ingredients

| | |
|---|---|
| Liquid paraffin | 247 g |
| Solid Paraffin | 133 g |
| Polysorbate 80® | 20 g |
| Salmon calcitonin | 330000 I.U. |

Preparation:

The oily vehicle is first prepared by mixing the solid paraffin with the liquid paraffin, followed by adding the surfactant. The calcitonin is then added to the oily vehicle, stirring until the mixture is homogeneous. The mass is then filled into the capsules.

BLOOD CALCIUM LEVEL LOWERING ACTIVITY TESTS

To the purpose of evaluating the calcitonin absorption in the various formulations, blood calcium level lowering activity tests have been carried out by using 36 male Sprague Dawley rats, divided in 4 groups which were in turn divided in 3 subgroups containing three rats each.

The animals of one group have been used as controls.

To the animals of one group a preparation containing 0.2 I.U./kg of calcitonin in a volume of 0.4 ml of water for injectable solutions per 100 g of rat body weight has been administered intravenously (i.v. formulation).

To the animals of two other groups the formulation of example 1 and 2, respectively, where administered in an amount of 0.5 I.U./kg of salmon calcitonin in a volume of 0.2 ml of vehicle per 100 g of rat body weight. For the administration of the rectal formulations, a rectal probe has been used.

30, 60 and 90 minutes after the administration, blood was withdrawn from each group.

The plasmatic calcium has been dosed by means of a spectrophotometer within 6 hours from the blood abstraction.

The results are shown in Table 1 hereinafter.

TABLE 1

| Formulation | Withdrawal time (min) | Blood calcium level (mean values in ppm) | % Diminution of plasmatic calcium |
|---|---|---|---|
| Control | 30 | 1.958±0.18 | // |
|  | 60 | 1.932±0.38 | // |
|  | 90 | 1.803±0.11 | // |
| i.v. | 30 | 1.656±0.09 | 15 |
|  | 60 | 1.738±0.09 | 10 |
|  | 90 | 1.526±0.07 | 15 |
| EXAMPLE 1 | 30 | 1.549±0.04 | 20 |
|  | 60 | 1.672±0.02 | 13 |
|  | 90 | 1.578±0·02 | 12 |
| EXAMPLE 2 | 30 | 1.562±0.05 | 20 |
|  | 60 | 1.633±0.01 | 15 |
|  | 90 | 1.620±0.03 | 10 |

The results in Table 1 above clearly show that the formulations for the rectal administration herein described have a calcium level lowering effect that can be compared with that obtained by means of the intravenous administration. The rectal administration clearly implies marked advantages relative to the intravenous one in that it is better manageable and easier to use.

STABILITY TESTS

To the purpose of evaluating the stability of the pharmaceutical composition described in the present invention, two stability tests for soft gelatine capsules containing the pharmaceutical composition described in Example 4 have been carried out. The stability tests have been carried out at 5°C and at 21°C for 12 months, during which the external features (colour and appearance) of the soft gelatine capsules have been assessed and the active ingredient contents (PPM) and its percent in the tested capsules has been assayed with known techniques, such as HPLC.

The obtained values are shown hereinafter in Table 2.

5

TABLE 2

| | Start (T zero) | | 2 Months | | 4 Months | | 6 Months | | 12 Months | |
|---|---|---|---|---|---|---|---|---|---|---|
| | 5°C | 21°C | 5°C | 21°C | 5°C | 21°C | 5°C | 21°C | 5°C | 21°C |
| COLOUR OF SOSPENSION | IVORY | IVORY | * | * | * | * | * | * | * | * |
| COLOUR OF CAPSULE | OCHRE | OCHRE | * | * | * | * | * | * | * | * |
| APPEARANCE OF SUSPENSION | PEARLY, HOMOGENEOUS | PEARLY, HOMOGENEOUS | * | * | * | * | * | * | * | * |
| APPEARANCE OF CAPSULE | TRANSPARENT, HOMOGENEOUS | TRANSPARENT, HOMOGENEOUS | * | * | * | * | * | * | * | * |
| HPLC ASSAY CALCITONIN P.P.M. % | 171 PPM 100 % | 171 PPM 100% | 173 PPM 101.1 % | 169 PPM 98.8 % | 163 PPM 95.3 % | 175 PPM 102.3% | 168 PPM 98.2% | 170 PPM 99.4% | 167.7 PPM 98.0% | 167 PPM 97.6% |

\* UNCHANGED

From the results in Table 2, it can be seen that the stability of the pharmaceutical compositions contained in the soft gelatine capsules is good even at a temperature of 21°C and for a sufficiently long time, thereby providing a valid support for the possible advanced therapeutical uses of said compositions.

**Claims**

**Claims for the following Contracting States : BE, CH, DE, FR, IT, LI**

1.  A pharmaceutical composition for rectal amdinistration containing calcitonin dispersed into paraffin characterized in that it consists of soft gelatin capsules containing from 400 to 2000 mg of a dispersion of from 50-600 I.U. of calcitonin into 400-2000 mg of an oily vehicle comprising a mixture of 40-90% of a liquid paraffin and 10-60% of at least a soft and/or solid paraffin.

2.  A pharmaceutical composition according to claim 1 characterized in that said soft gelatin capsules contain from 900 to 1200 mg of said dispersion.

3.  A pharmaceutical composition according to claims 1 and 2, characterized in that said calcitonin is a salmon calcitonin.

4.  A pharmaceutical composition according to claims 1 to 3, characterized in that said calcitonin is present in an amount of from 50 to 300 I.U.

5.  A pharmaceutical composition according to claims 1 to 4, characterized in that said calcitonin, is dispersed in 900 mg of said oily vehicle.

6.  A pharmaceutical composition according to claims 1 to 5, characterized in that said oily vehicle consists of a mixture of 65% of liquid paraffin and 35% of soft paraffin and/or solid paraffin.

7.  A pharmaceutical composition according to claim 1 to 6, characterized in that it contains 10-50 mg of at least one non-ionic surfactant.

**Claims for the following Contracting State : ES**

1.  A process for preparing a pharmaceutical composition for rectal administration containing calcitonin dispersed into paraffin characterized in that soft gelatin capsules are filled with from 400 to 2000 mg of a dispersion of from 50-600 I.U. of calcitonin into 400-2000 mg of an oily vehicle comprising a mixture of 40-90% of a liquid paraffin and 10-60% of at least a soft and/or solid paraffin.

2.  A process according to claim 1 characterized in that said soft gelatin capsules contain from 900 to 1200 mg of said dispersion.

3.  A process according to claims 1 and 2, characterized in that said calcitonin is a salmon calcitonin.

4.  A process according to claims 1 to 3, characterized in that said calcitonin is present in an amount of from 50 to 300 I.U.

5.  A process according to claims 1 to 4, characterized in that said calcitonin, is dispersed in 900 mg of said oily vehicle.

6.  A process according to claims 1 to 5, characterized in that said oily vehicle consists of a mixture of 65% of liquid paraffin and 35% of soft paraffin and/or solid paraffin.

7.  A process according to claim 1 to 6, characterized in that it contains 10-50 mg of at least one non-ionic surfactant.

**Patentansprüche**

**Patentansprüche für folgende Vertragsstaaten : BE, CH, DE, FR, IT, LI**

1.  Arzneimittel zur rektalen Verabreichung, enthaltend in Paraffin dispergiertes Calcitonin, dadurch **gekennzeichnet,** daß es aus Weichgelatine-Kapseln besteht, die 400 bis 2000 mg einer Dispersion aus 50-600 I.E. Calcitonin in 400-2000 mg eines öligen, ein Gemisch aus 40-90% eines flüssigen Paraffins und 10-60% von mindestens einem weichen und/oder festen Paraffin umfassenden Trägers enthalten.

2. Arzneimittel nach Anspruch 1, dadurch **gekennzeichnet**, daß die Weichgelatine-Kapseln 900 bis 1200 mg der Dispersion enthalten.

3. Arzneimittel nach Anspruch 1 und 2, dadurch **gekennzeichnet**, daß das Calcitonin ein Lachs-Calcitonin ist.

4. Arzneimittel nach Ansprüchen 1 bis 3, dadurch **gekennzeichnet**, daß das Calcitonin in einer Menge von 50 bis 300 I.E. vorhanden ist.

5. Arzneimittel nach Ansprüchen 1 bis 4, dadurch **gekennzeichnet**, daß das Calcitonin in 900 mg des öligen Trägers dispergiert ist.

6. Arzneimittel nach Ansprüchen 1 bis 5, dadurch **gekennzeichnet**, daß der ölige Träger aus einem Gemisch von 65% flüssigem Paraffin und 35% weichem Paraffin und/oder festem Paraffin besteht.

7. Arzneimittel nach Ansprüchen 1 bis 6, dadurch **gekennzeichnet**, daß es 10-50 mg von mindestens einem nicht-ionischen grenzflächenaktiven Mittel enthält.

**Patentansprüche für folgenden Vertragsstaat : ES**

1. Verfahren zur Herstellung eines Arzneimittel zur rektalen Verabreichung, enthaltend in Paraffin dispergiertes Calcitonin, dadurch **gekennzeichnet**, daß Weichgelatine-Kapseln mit 400 bis 2000 mg einer Dispersion aus 50-600 I.E. Calcitonin in 400-2000 mg eines öligen, ein Gemisch aus 40-90% eines flüssigen Paraffins und 10-60% von mindestens einem weichen und/oder festen Paraffin umfassenden Trägers aufgefüllt werden.

2. Verfahren nach Anspruch 1, dadurch **gekennzeichnet**, daß die Weichgelatine-Kapseln 900 bis 1200 mg der Dispersion enthalten.

3. Verfahren nach Anspruch 1 und 2, dadurch **gekennzeichnet**, daß das Calcitonin ein Lachs-Calcitonin ist.

4. Verfahren nach Ansprüchen 1 bis 3, dadurch **gekennzeichnet**, daß das Calcitonin in einer Menge von 50 bis 300 I.E. vorhanden ist.

5. Verfahren nach Ansprüchen 1 bis 4, dadurch **gekennzeichnet**, daß das Calcitonin in 900 mg des öligen Trägers dispergiert ist.

6. Verfahren nach Ansprüchen 1 bis 5, dadurch **gekennzeichnet,** daß der ölige Träger aus einem Gemisch von 65% flüssigem Paraffin und 35% weichem Paraffin und/oder festem Paraffin besteht.

7. Verfahren nach Ansprüchen 1 bis 6, dadurch **gekennzeichnet**, daß 10-50 mg von mindestens einem nicht-ionischen grenzflächenaktiven Mittel enthalten sind.

**Revendications**

**Revendications pour les Etats contractants suivants : BE, CH, DE, FR, IT, LI**

1. Composition pharmaceutique pour l'administration rectale contenant de la calcitonine dispersée dans de la paraffine, caractérisée en ce qu'elle est constituée de capsules de gélatine molle contenant de 400 à 2000 mg d'une dispersion de 50 à 600 U.I. de calcitonine dans 400-2000 mg d'un véhicule huileux comprenant un mélange de 40-90 % d'une paraffine liquide et de 10-60 % d'au moins une paraffine molle et/ou solide.

2. Composition pharmaceutique selon la revendication 1, caractérisée en ce que lesdites capsules de gélatine molle contiennent de 900 à 1200 mg de ladite dispersion.

3. Composition pharmaceutique selon les revendications 1 et 2, caractérisée en ce que ladite calcitonine est une calcitonine de saumon.

4. Composition pharmaceutique selon les revendications 1 à 3, caractérisée en ce que ladite calcitonine est présente dans une quantité de 50 à 300 U.I.

5. Composition pharmaceutique selon les revendications 1 à 4, caractérisée en ce que ladite calcitonine est dispersée dans 900 mg dudit véhicule huileux.

6. Composition pharmaceutique selon les revendications 1 à 5, caractérisée en ce que ledit véhicule huileux se compose d'un mélange de 65 % de paraffine liquide et de 35 % de paraffine molle et/ou de paraffine solide.

7. Composition pharmaceutique selon les revendications 1 à 6, caractérisée en ce qu'elle contient 10 à 50 mg d'au moins un agent tensio-actif non-ionique.

**Revendications pour l'Etat contractant suivant : ES**

1. Procedé pour la préparation d'une composition pharmaceutique pour l'administration rectale contenant de la calcitonine dispersée dans de la paraffine, caractérisée en ce qu'elle est constituée de capsules de gélatine molle contenant de 400 à 2000 mg d'une dispersion de 50 à 600 U.I. de calcitonine dans 400-2000 mg d'un véhicule huileux comprenant un mélange de 40-90 % d'une paraffine liquide et de 10-60 % d'au moins une paraffine molle et/ou solide.

2. Procedé selon la revendication 1, caractérisée en ce que lesdites capsules de gélatine molle contiennent de 900 à 1200 mg de ladite dispersion.

3. Procedé selon les revendications 1 et 2, caractérisée en ce que ladite calcitonine est une calcitonine de saumon.

4. Procedé selon les revendications 1 à 3, caractérisée en ce que ladite calcitonine est présente dans une quantité de 50 à 300 U.I.

5. Procedé selon les revendications 1 à 4, caractérisée en ce que ladite calcitonine est dispersée dans 900 mg dudit véhicule huileux.

6. Procedé selon les revendications 1 à 5, caractérisée en ce que ledit véhicule huileux se compose d'un mélange de 65 % de paraffine liquide et de 35 % de paraffine molle et/ou de paraffine solide.

7. Procedé selon les revendications 1 à 6, caractérisée en ce qu'elle contient 10 à 50 mg d'au moins un agent tensio-actif non-ionique.